# EUROPEAN PATENT SPECIFICATION

(11) **EP 2 363 088 B1**
(45) Date of publication and mention of the grant of the patent: **19.09.2012**
(21) Application number: 11001689.6
(22) Date of filing: 01.03.2011
(51) Int. Cl.: A61B 18/18

(54) **Sensors on patient side for a microwave generator**
Sensoren auf Patientenseite für einen Mikrowellengenerator
Capteurs positionnés sur le côté du patient pour générateur de micro-ondes

(30) Priority: 01.03.2010 US 715212
(43) Date of publication of application: 07.09.2011
(73) Proprietor: Vivant Medical, Inc., Boulder CO 80301 (US)
(72) Inventor: Behnke, Robert J., Erie, CO 80516 (US)
(74) Representative: Schmidt, Steffen

(56) References cited:
- EP-A1- 2 014 249
- WO-A1-2008/044012
- US-A- 6 067 475

## Description

### BACKGROUND

### 1. Technical Field

The present disclosure relates generally to microwave ablation procedures that utilize microwave surgical devices having a microwave antenna which may be inserted directly into tissue for diagnosis and treatment of diseases. More particularly, the present disclosure is directed to measuring tissue impedance during a microwave ablation procedure.

### 2. Background of the Related Art

In the treatment of diseases such as cancer, certain types of cancer cells have been found to denature at elevated temperatures (which are slightly lower than temperatures normally injurious to healthy cells.) These types of treatments, known generally as hyperthermia therapy, typically utilize electromagnetic radiation to heat diseased cells to temperatures above 41°C, while maintaining adjacent healthy cells at lower temperatures where irreversible cell destruction will not occur. Other procedures utilizing electromagnetic radiation to heat tissue also include ablation and coagulation of the tissue. Such ablation procedures, e.g., such as those performed for menorrhagia, are typically done to ablate and coagulate the targeted tissue to denature or kill the tissue. Many procedures and types of devices utilizing electromagnetic radiation therapy are known in the art. Such microwave therapy is typically used in the treatment of tissue and organs such as the prostate, heart, liver, lung, kidney, and breast.

One non-invasive procedure generally involves the treatment of tissue (e.g., a tumor) underlying the skin via the use of microwave energy. The microwave energy is able to non-invasively penetrate the skin to reach the underlying tissue. However, this non-invasive procedure may result in the unwanted heating of healthy tissue. Thus, the non-invasive use of microwave energy requires a great deal of control.

Presently, there are several types of microwave probes in use, e.g., monopole, dipole, and helical. One type is a monopole antenna probe, which consists of a single, elongated microwave conductor exposed at the end of the probe. The probe is typically surrounded by a dielectric sleeve. The second type of microwave probe commonly used is a dipole antenna, which consists of a coaxial construction having an inner conductor and an outer conductor with a dielectric junction separating a portion of the inner conductor. The inner conductor may be coupled to a portion corresponding to a first dipole radiating portion, and a portion of the outer conductor may be coupled to a second dipole radiating portion. The dipole radiating portions may be configured such that one radiating portion is located proximally of the dielectric junction, and the other portion is located distally of the dielectric junction. In the monopole and dipole antenna probe, microwave energy generally radiates perpendicularly from the axis of the conductor.

The typical microwave antenna has a long, thin inner conductor that extends along the axis of the probe and is surrounded by a dielectric material and is further surrounded by an outer conductor around the dielectric material such that the outer conductor also extends along the axis of the probe. In another variation of the probe that provides for effective outward radiation of energy or heating, a portion or portions of the outer conductor can be selectively removed. This type of construction is typically referred to as a "leaky waveguide" or "leaky coaxial" antenna. Another variation on the microwave probe involves having the tip formed in a uniform spiral pattern, such as a helix, to provide the necessary configuration for effective radiation. This variation can be used to direct energy in a particular direction, e.g., perpendicular to the axis, in a forward direction (i.e., towards the distal end of the antenna), or combinations thereof.

Invasive procedures and devices have been developed in which a microwave antenna probe may be either inserted directly into a point of treatment via a normal body orifice or percutaneously inserted. Such invasive procedures and devices potentially provide better temperature control of the tissue being treated. Because of the small difference between the temperature required for denaturing malignant cells and the temperature injurious to healthy cells, a known heating pattern and predictable temperature control is important so that heating is confmed to the tissue to be treated. For instance, hyperthermia treatment at the threshold temperature of about 41.5°C generally has little effect on most malignant growth of cells. However, at slightly elevated temperatures above the approximate range of 43°C to 45°C, thermal damage to most types of normal cells is routinely observed. Accordingly, great care must be taken not to exceed these temperatures in healthy tissue.

In order to achieve better control during ablation procedures, sensors are utilized to measure and determine various properties of the affected tissue region and/or the ablation system. Typically, such sensors tend to be on the ground reference side and are not referenced to the patient. Accordingly, the accuracy of the sensors can be compromised due to the isolation boundary from the ground referenced side to the patient, especially if phase is being measured. Even the type of isolation boundary may cause inaccuracies in sensor readings. For instance, if capacitors are used as the isolation boundary, the tolerance and/or mechanical mounting of the capacitors may cause phase and impedance variations that affect the sensors. If a coupling mechanism is used as the isolation boundary, it may be difficult to have a repeatable coupling from device to device thereby leading to inaccurate sensor readings.

Document EP-A-2014249 discloses the most relevant prior art.

### SUMMARY

The present disclosure, relates to an electrosurgical system provided for use in electrosurgical procedures. The system includes a generator configured to provide electrosurgical energy to an instrument and a dual directional coupler coupled between the generator and the instrument and configured to sample the electrosurgical energy. The system also includes a sensor module configured to detect one or more properties of the sampled electrosurgical energy, a controller configured to receive the detected one or more properties and configured to control the output of the generator based on the detected one or more properties, and an isolation device coupled between the sensor module and the controller.

The sensor module detects at least one of a phase, forward voltage or reflected voltage. The isolation device may include one or more capacitors or one or more transformers.

In another embodiment, the electrosurgical system may include an analog to digital converter operatively coupled between the sensor module and the isolation device where the isolation device may be an opto-coupler.

### BRIEF DESCRIPTION OF THE DRAWINGS

The above and other aspects, features, and advantages of the present disclosure will become more apparent in light of the following detailed description when taken in conjunction with the accompanying drawings in which:

Fig. 1 is a schematic block diagram of an electrosurgical system according to an embodiment of the present disclosure; and

Fig. 2 is a schematic block diagram of an electrosurgical system according to another embodiment of the present disclosure. The embodiment shown in figure 2 is not part of the invention.

### DETAILED DESCRIPTION

Particular embodiments of the present disclosure are described hereinbelow with reference to the accompanying drawings; however, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure. Like reference numerals may refer to similar or identical elements throughout the description of the figures.

Electromagnetic energy is generally classified by increasing energy or decreasing wavelength into radio waves, microwaves, infrared, visible light, ultraviolet, X-rays and gamma-rays. As used herein, the term "microwave" generally refers to electromagnetic waves in the frequency range of 300 megahertz (MHz) (3 x 10⁸ cycles/second) to 300 gigahertz (GHz) (3 x 10¹¹ cycles/second). As used herein, the term "RF" generally refers to electromagnetic waves having a lower frequency than microwaves. The phrase "ablation procedure" generally refers to any ablation procedure, such as RF or microwave ablation or microwave ablation assisted resection. The phrase "transmission line" generally refers to any transmission medium that can be used for the propagation of signals from one point to another.

The present disclosure is directed to an electrosurgical system for use in performing a medical procedure. Such a system is described in document EP-A-2014249.

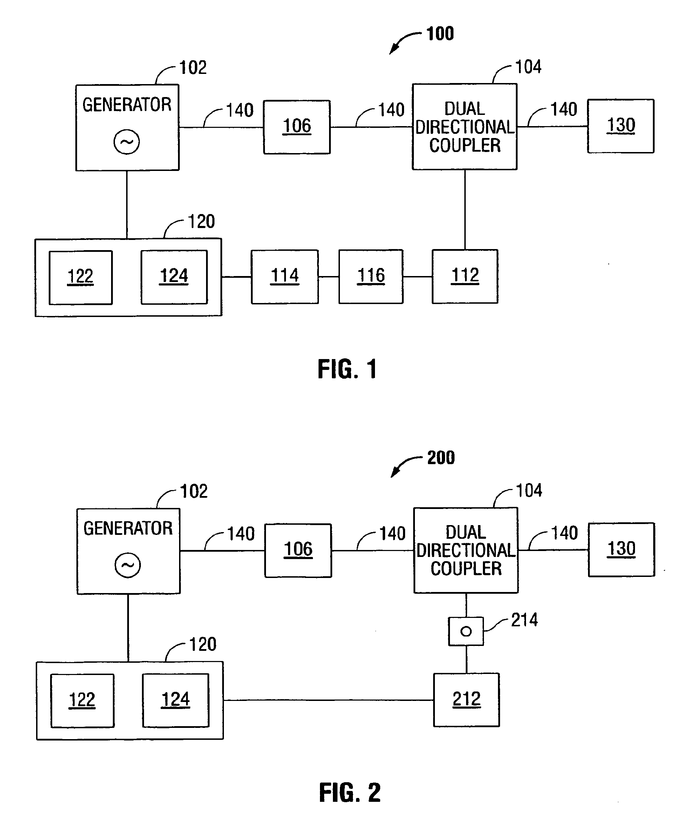

Fig. 1 is a schematic block diagram of an electrosurgical system 100 according to an embodiment of the present disclosure. System 100 may include a generator 102 that supplies electromagnetic energy, e.g., microwave of RF energy, to an energy delivering implement or instrument 130 (e.g., an electrosurgical pencil, a microwave ablation antenna, etc.) via a transmission line 140 (e.g., a coaxial cable) or the like.

Not explicitly shown in Fig. 1, the generator 102 includes suitable input controls (e.g., buttons, activators, switches, touch screen, etc.) for controlling the generator 102, as well as one or more display screens for providing the surgeon with variety of output information (e.g., intensity settings, treatment complete indicators, etc.). The controls allow the surgeon to adjust power of the energy, waveform, and other parameters to achieve the desired waveform suitable for a particular task (e.g., tissue ablation). Further, the instrument 130 may include a plurality of input controls which may be redundant with certain input controls of the generator 102. Placing the input controls at the instrument 130 allows for easier and faster modification of energy parameters during the surgical procedure without requiring interaction with the generator 102.

System 100 also includes a dual directional coupler 104 operatively coupled between generator 102 and instrument 130. Dual directional coupler 104 is used to sample the forward and reflected energy in system 100. An isolation device 106 is provided between generator 102 and dual directional coupler 104 to isolate the generator 102 from a patient. Isolation device may be discrete components such as a capacitor(s) or transformer or it may be built into transmission line 140.

Forward energy and reflected energy sampled by dual directional coupler 104 is provided to sensor module 112 via transmission lines 108 and 110, respectively. The sensor module 112 may include a plurality of sensors (not explicitly shown) strategically located for sensing various properties or conditions, e.g., tissue impedance, voltage at the tissue site, current at the tissue site, etc. The sensors are provided with leads (or wireless) for transmitting information to the controller 120. The sensor module 112 may include control circuitry that receives information from multiple sensors, and provides the information and the source of the information (e.g., the particular sensor providing the information) to the controller 120.

More particularly, the sensor module 112 may include a real-time voltage sensing system (not explicitly shown) and a real-time current sensing system (not explicitly shown) for sensing real-time values related to applied voltage and current at the surgical site. Additionally, an RMS voltage sensing system (not explicitly shown) and an RMS current sensing system (not explicitly shown) may be included for sensing and deriving RMS values for applied voltage and current at the surgical site.

The controller 120 includes a microprocessor 122 having a memory 124 which may be volatile type memory (e.g., RAM) and/or non-volatile type memory (e.g., flash media, disk media, etc.). The microprocessor 122 includes an output port connected to the generator 102 that allows the microprocessor 122 to control the output of the generator 102 according to either open and/or closed control loop schemes.

A closed loop control scheme generally includes a feedback control loop wherein the sensor module 112 provides feedback to the controller 120 (i.e., information obtained from one or more sensing mechanisms for sensing various parameters such as tissue impedance, tissue temperature, forward and reflected current and/or voltage, etc.). The controller 120 then signals the generator 102 which then adjusts the output electromagnetic energy. The controller 120 also receives input signals from the input controls of the generator 102 and/or instrument 130. The controller 120 utilizes the input signals to adjust the power output of the generator 102 and/or instructs the generator 102 to perform other control functions.

The microprocessor 122 is capable of executing software instructions for processing data received by the sensor module 112, and for outputting control signals to the generator 102, accordingly. The software instructions, which are executable by the controller 120, are stored in the memory 124 of the controller 120.

Sensor module 112 provides a signal that is indicative of the various sensed properties or conditions to an analog to digital converter (ADC) 116 to convert the signal to a digital signal. The digital signal is sent to isolation device 114 which conveys the signal to controller 120. Isolation device 114 may be an opto-coupler used to transmit information or data from the patient side to the generator side. Sensor module 112 is on the patient side and may be configured to convert the signal to digital to go across the isolation boundary provided by isolation device 114.

The controller 120 may include analog and/or logic circuitry for processing the sensed values and determining the control signals that are sent to the generator 102, rather than, or in combination with, the microprocessor 122.

An isolation device 114 is coupled between sensor module 112 and controller 120. Isolation device 114 provides electrical isolation between the patient and generator 102. By moving the sensors to the patient side, the interaction between dual directional coupler 104 and the isolation boundary is removed. This allows for a more accurate measurement of phase, forward and reflected voltages. Additionally, any tolerance issue with the isolation boundary does not affect the sensors. Isolation device 114 may be a capacitor, opto-coupler or a transformer.

It should be noted that the components of system 100 described hereinabove may be included in a single device or may be separate components that are operatively connected to each other.

Turning to Fig. 2, an electrosurgical system according to another embodiment of the present disclosure is shown generally as 200. Electrosurgical system 200 includes components similar to electrosurgical system 100 which are described in more detail above and will not be repeated hereinbelow. Similar to sensor module 112, sensor module 212 may include a plurality of sensors (not explicitly shown) strategically located for sensing various properties or conditions, e.g., tissue impedance, voltage at the tissue site, current at the tissue site, etc. System 200 uses an isolation device 214, such as a capacitor or transformer, to transfer the dual directional coupler signals to the reference of the generator. The isolation device 214 is provided between dual directional coupler 104 and sensor module 212. The sensor module 212 no longer needs to convert the signal to digital as in system 100. However, sensor module 212 may digitize the signal if desired or necessary.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figs. are presented only to demonstrate certain examples of the disclosure. Other elements, steps, methods and techniques that are insubstantially different from those described above and/or in the appended claims are also intended to be within the scope of the disclosure.

## Claims

1. An electrosurgical system (100), comprising:
a generator (102) configured to provide electrosurgical energy to an instrument;
a dual directional coupler (104) coupled between the generator and the instrument and configured to sample the electrosurgical energy;
a sensor module (112) configured to detect at least one property of the sampled electrosurgical energy;
a controller (120) configured to receive the detected at least one property and configured to control the output of the generator based on the detected at least one property; **characterized by**
an isolation device (114) coupled between the sensor module and the controller.

2. The electrosurgical system according to claim 1, wherein the sensor module detects at least one of a phase, forward voltage or reflected voltage.

3. The electrosurgical system according to claim 1, wherein the isolation device includes at least one capacitor.

4. The electrosurgical system according to claim 1, wherein the isolation device includes at least one transformer.

5. The electrosurgical system according to claim 1, further comprising an analog to digital converter operatively coupled between the sensor module and the isolation device.

6. The electrosurgical system according to claim 5, wherein the isolation device includes at least one opto-coupler.

## Patentansprüche

1. Elektrochirurgisches System (100) mit:
einem Generator (102), der dazu ausgebildet ist, einem Instrument elektrochirurgische Energie zuzuführen;
einem Zweifach-Richtkoppler (104), der zwischen den Generator und das Instrument gekoppelt und dazu ausgebildet ist, die elektrochirurgische Energie abzutasten;
einem Sensormodul (112), das dazu ausgebildet ist, mindestens eine Eigenschaft der abgetasteten elektrochirurgischen Energie zu erfassen;
einer Steuerung (120), die dazu ausgebildet ist, die erfasste mindestens eine Eigenschaft zu empfangen, und dazu ausgebildet ist, die Ausgangsleistung des Generators auf der Grundlage der erfassten mindestens einen Eigenschaft zu steuern; **gekennzeichnet durch**
eine Isoliervorrichtung (114), die zwischen das Sensormodul und die Steuerung gekoppelt ist.

2. Elektrochirurgisches System nach Anspruch 1, bei dem das Sensormodul mindestens eine Phase und/oder Vorwärtsspannung und/oder reflektierte Spannung erfasst.

3. Elektrochirurgisches System nach Anspruch 1, bei dem die Isoliervorrichtung mindestens einen Kondensator aufweist.

4. Elektrochirurgisches System nach Anspruch 1, bei dem die Isoliervorrichtung mindestens einen Transformator aufweist.

5. Elektrochirurgisches System nach Anspruch 1, ferner mit einem Analog-DigitalWandler, der funktionsfähig zwischen das Sensormodul und die Isoliervorrichtung gekoppelt ist.

6. Elektrochirurgisches System nach Anspruch 5, bei dem die Isoliervorrichtung mindestens einen Optokoppler aufweist.

## Revendications

1. Système électro-chirurgical (100) comprenant :
un générateur (102) configuré pour délivrer une énergie électro-chirurgicale à un instrument ;
un coupleur bidirectionnel (104) couplé entre le générateur et l'instrument et configuré pour échantillonner l'énergie électro-chirurgicale ;
un module de capteur (112) configuré pour détecter au moins une propriété de l'énergie électro-chirurgicale échantillonnée ;
un contrôleur (120) configuré pour recevoir l'au moins une propriété détectée et configuré pour commander la sortie du générateur sur la base de l'au moins une propriété détectée ; **caractérisé par**
un dispositif d'isolation (114) couplé entre le module de capteur et le contrôleur.

2. Système électro-chirurgical selon la revendication 1, dans lequel le module de capteur détecte au moins une d'une phase, d'une tension directe ou d'une tension réfléchie.

3. Système électro-chirurgical selon la revendication 1, dans lequel le dispositif d'isolation inclut au moins un condensateur.

4. Système électro-chirurgical selon la revendication 1, dans lequel le dispositif d'isolation inclut au moins un transformateur.

5. Système électro-chirurgical selon la revendication 1, comprenant en outre un convertisseur analogique à numérique opérationnellement couplé entre le module de capteur et le dispositif d'isolation.

6. Système électro-chirurgical selon la revendication 5, dans lequel le dispositif d'isolation inclut au moins un opto-coupleur.
